(19) Europäisches Patentamt

European Patent Office

Office européen des brevets

(11) Publication number: **0 471 205 A1**

# EUROPEAN PATENT APPLICATION

(21) Application number: **91112109.3**

(22) Date of filing: **19.07.91**

(51) Int. Cl.5: **C12N 5/20**, C12P 21/08, G01N 33/574, G01N 33/577, A61K 47/48, A61K 43/00, A61K 39/395, C07K 3/18, A61K 49/02

(30) Priority: **15.08.90 US 567984**

(43) Date of publication of application:
**19.02.92 Bulletin 92/08**

(84) Designated Contracting States:
**AT BE CH DE DK ES FR GB GR IT LI LU NL SE**

(71) Applicant: **E.R. SOUIBB & SONS, INC.**
**P.O.Box 4000**
**Princeton New Jersey 08543-4000(US)**

(72) Inventor: **Eager, Kendra B.**
**702 Windsor Commons**
**Cranbury, NJ 08512(US)**

(74) Representative: **Josif, Albert et al**
**Baaderstrasse 3**
**W-8000 München 5(DE)**

(54) Monoclonal antibodies which bind TRK proto-oncogene protein.

(57) Monoclonal antibodies which bind *trk* proto-oncogene protein and/or *trk* related oncogene protein, hybrid cell lines which produce these monoclonal antibodies, and methods for using these monoclonal antibodies.

EP 0 471 205 A1

The fusion of mouse myeloma cells to spleen cells derived from immunized mice by Kohler and Milstein in 1975 [Nature 256, 495-497 (1975)] demonstrated, for the first time, that it was possible to obtain continuous cell lines making homogeneous (so-called "monoclonal") antibodies. Since this seminal work, much effort has been directed to the production of various hybrid cell lines (also called "hybridomas") and to the use of the antibodies made by these hybridomas for various scientific investigations. While the general technique for the preparation of hybridomas and monoclonal antibodies is well-known, there are many difficulties met and variations required for each specific case. In fact, there is no assurance, prior to attempting to prepare a given hybridoma, that the desired hybridoma will be obtained, that it will produce antibody if obtained, or that the antibody so produced will have the desired specificity.

The *trk* oncogene (tropomyosin receptor kinase) was first identified in a human colon carcinoma biopsy by a gene transfer assay. The *trk* oncogene is a member of the large family of tyrosine kinase genes. It was found that the tyrosine kinase receptor moiety was fused to non-muscle tropomyosin [Martin-Zanca et al., Nature 319, 743-748 (1986); Mitra et al., Proc. Natl. Acad. Sci. USA 84, 6707-6711 (1987)] and this somatic rearrangement activated the *trk* proto-oncogene [Coulier et al., Molec. Cell. Biol. 9, 15-23 (1989)]. Other cellular sequences have been shown to be responsible for the activation of the *trk* oncogene [Kozma et al., EMBO J. 7, 147-154 (1988); Coulier et al., supra; Ziemiecki et al., EMBO J. 4, 191-196 (1990)]. Spontaneous generation of *trk* oncogenes has been shown to occur as a consequence of transfection of NIH3T3 cells with the *trk* proto-oncogene [Oskam et al., Proc. Natl. Acad. Sci. USA 85, 2964-2968 (1988)].

The *trk* proto-oncogene protein is comprised of 790 amino acids and much of the extracellular domain of this product has been substituted by non-muscle tropomyosin during oncogene activation [Martin-Zanca et al., Molec. Cell. Biol. 9, 24-33 (1989)]. The human *trk* proto-oncogene encodes a polypeptide of 80,000 daltons and contains numerous potential sites for N-glycoslyation. It is believed that the 110 kilodalton glycoprotein of proto-*trk* is the primary translational product and additional glycosylations give rise to the mature *trk* proto-oncogene protein of 140 kilodaltons. The *trk* proto-oncogene protein is highly homologous to the originally isolated *trk* oncogene protein with the exception of the first 392 amino acids which contain numerous consensus sequences for N-linked glycosylation. The transmembrane domain, tyrosine kinase catalytic domain and cytoplasmic domain are identical between the *trk* proto-oncogene protein and the *trk* oncogene protein. A murine gene, designated *trk*B, has been identified which is structurally similar to the human *trk* proto-oncogene [Klein, et al., EMBO J. 8, 3701-3709 (1989)].

Polyclonal antisera have been produced [Martin-Zanca et al., Mol. Cell. Biol. 9, 24-33 (1989)] which recognize the catalytic domain but are unable to distinguish the *trk* oncogene protein and proto-oncogene protein. A group of monoclonal antibodies specific to the *trk* proto-oncogene protein would be useful in understanding the function of the carbohydrate-rich extracellular domain, in addition to using such antibodies to examine the distribution of the *trk* proto-oncogene protein in human tissues.

The present invention aids in solving these and other needs in the art.

The present invention concerns hybrid cell lines which produce monoclonal antibodies which bind *trk* proto-oncogene protein and/or *trk* related oncogene protein.

The present invention further concerns monoclonal antibodies which bind *trk* proto-oncogene protein and/or *trk* related oncogene protein.

The present invention additionally concerns immunoassay methods for detecting the presence of *trk* proto-oncogene protein and/or *trk* related oncogene protein in a sample.

The present invention also concerns immunoassay methods for quantitatively determining the amount of *trk* proto-oncogene protein and/or *trk* related oncogene protein in a sample.

The present invention further concerns methods for the purification of *trk* proto-oncogene protein and/or *trk* related oncogene protein using monoclonal antibodies which bind *trk* proto-oncogene protein and/or *trk* related oncogene protein.

The present invention concerns hybrid cell lines, also called hybridomas, monoclonal antibodies and immunoassay methods utilizing these antibodies.

In particular, the present invention concerns hybrid cell lines which produce monoclonal antibodies which bind *trk* proto-oncogene protein and/or *trk* related oncogene protein.

As used in the present application, the term "*trk* proto-oncogene protein" means the protein encoded by the *trk* proto-oncogene. The term "*trk* related oncogene protein" means a rearranged *trk* proto-oncogene protein having the ability to transform cells and having one or more epitopes from the extracellular domain of the *trk* proto-oncogene protein.

As used in this application, the phrase "*trk* proto-oncogene protein and/or *trk* related oncogene protein" means only *trk* proto-oncogene protein, or only *trk* related oncogene protein, or both *trk* proto-oncogene protein and *trk* related oncogene protein.

Particularly preferred is the hybrid cell line designated as TTM7-41.1, which is a subclone of TTM7-41, or hybrid cell lines which have the identifying characteristics of this hybrid cell line.

Hybrid cell line TTM7-41.1 was deposited with the American Type Culture Collection, Rockville, Maryland on April 19, 1990 under the Budapest Treaty and assigned ATCC accession no. HB 10432.

Also preferred are the hybrid cell lines designated as TTM1-8, TTM6-13, TTM6-46, TTM6-50, TTM7-4, TTM7-41, TTM9-9, TTM9-12, TUM5-47 or TUM7-9, or subclones derived therefrom, or hybrid cell lines which have the identifying characteristics of these hybrid cell lines.

The hybrid cell lines of the present invention may be produced by various methods generally known to those of ordinary skill in the art. In general, the method involves immunizing suitable mammals, for example mice, with the antigen of interest, in this case *trk* proto-oncogene protein, fusing antibody producing cells isolated from the animal with myeloma cells, cloning the resulting hybrid cells and selecting those cells which produce the desired monoclonal antibody which binds the antigen of interest.

The usual mammals used for immunizations are mice, especially BALB/c mice, but other mammals and mouse strains may also be employed. Immunizations may be performed by the subcutaneous administration of recombinant host cells expressing *trk* proto-oncogene protein, in which case the immunized mammals are stimulated continuously as the cell mass grows, or with purified *trk* proto-oncogene protein. If purified *trk* proto-oncogene protein is used, immunizations are performed in a manner known in the art, such as by administering parenterally, intraperitoneally, intravenously and/or subcutaneously, three to six injections each containing an appropriate amount of antigen (i.e, from about 1 $\mu$g to about 50 $\mu$g), at intervals of about one to six weeks, usually together with an adjuvant, for example, complete or incomplete Freund's adjuvant. While immunizations are generally performed in vivo, various in vitro procedures are also known and may be employed.

Antibody-producing cells of the immunized animals, usually spleen cells, are taken from the animals and fused with myeloma cells of a suitable cell line. Myeloma cell lines and cell lines derived therefrom are known as suitable fusion partners. The myeloma cell line is generally derived from the same species as the immunized mammal, since intra-species hybrids are more viable than inter-species hybrids. Myeloma cells that lack the enzyme hypoxanthine-guaninephosphoribosyl transferase (HGPRT) or the enzyme thymidine kinase (TK) and that, for that reason, do not survive in a selective culture medium containing hypoxanthine, aminopterin and thymidine (HAT medium), may be employed. Myeloma cells and cell lines prepared therefrom that do not survive in HAT medium and do not secrete any immuno-globulins or parts thereof, for example, cell lines P3X63-Ag8.653 and Sp2/0-Ag14, may also be used. Various fusion-promoters, for example, Sendai virus or other paramyxoviruses, optionally in UV-inactivated form, calcium ion, surface-active lipids, such as isolecithin, or polyethylene glycol may also be employed. Myeloma cells are usually fused with a three- to twenty-fold excess of spleen cells from immunized animals in a solution containing from 30 to 50% polyethylene glycol (PEG) having a molecular weight of about 1000 to 4000. Exposure to PEG for about 2 to 3 minutes appears to be optimal to prevent toxicity to cells; temperatures of about 37° are recommended.

After fusion, the cells are partitioned out and cultured in selective HAT medium, with only hybrid cells surviving, since these combine, from the myeloma cells, the ability to grow in vitro and, from the antibody-producing cells of the immunized animals, the missing HGPRT or TK genes and, therewith, the ability to survive in HAT medium.

Suitable culture media for the growth of the hybridoma cells are the customary standard culture media, for example, Dulbecco's Modified Eagles Medium or Roswell Park Memorial Institute (RPMI) 1640 medium containing 10-15% fetal calf serum, supplemented with antibiotics. At the beginning of cell growth, so-called feeder cells, for example normal mouse peritoneal exudate cells, spleen cells, bone marrow macrophages or the like, may be added. At regular intervals, said culture media may be supplemented by selective HAT medium to prevent hybrid cells from being overgrown by ordinary myeloma cells still present after the initial HAT selection process.

The cell culture supernatants of the hybrid cells surviving HAT selection are examined for the presence of the desired monoclonal antibodies. Advantageously, the cell supernatants are tested in an immunoassay, for example, radioimmunoassay or enzyme immunoassay, that demonstrates the binding of monoclonal antibodies to the antigen of interest.

Those hybrid cells which produce antibodies having the desired specificity as well as other desirable characteristics can then be maintained as viable cultures and/or frozen for storage.

The present invention further concerns monoclonal antibodies which bind *trk* proto-oncogene protein and/or *trk* related oncogene protein.

Preferred are the monoclonal antibodies designated as MabTTM1-8, MabTTM6-13, MabTTM6-46, MabTTM6-50, MabTTM7-4, MabTTM7-41, MabTTM9-9, MabTTM9-12, MabTUM5-47 or MabTUM7-9, or monoclonal antibodies with the identifying characteristics of these monoclonal antibodies.

Particularly preferred is the monoclonal antibody designated as MabTTM7-41.1, or monoclonal antibodies with the identifying characteristics of this monoclonal antibody.

Also preferred are substantially purified monoclonal antibodies which bind *trk* proto-oncogene protein and/or *trk* related oncogene protein.

Additionally preferred are derivatives of monoclonal antibodies which bind *trk* proto-oncogene protein and/or *trk* related oncogene protein.

The monoclonal antibodies of the present invention may be produced by various methods generally known to those of ordinary skill in the art. Hybrid cells producing such antibodies may be cultured in vitro and the monoclonal antibodies isolated from the culture supernatants, or may be multiplied in vivo in a suitable mammal, and the monoclonal antibodies isolated from the body fluids of that mammal. If desired, a monoclonal antibody resulting from either of these techniques may be converted into a derivative thereof.

Suitable culture media for in vitro culturing are the customary standard culture media, for example, Dulbecco's Modified Eagles Medium or PPMI 1640 medium containing 10 to 15% fetal calf serum and supplemented with antibiotics.

Large quantities of the desired monoclonal antibodies may also be obtained by multiplying the hybrid cells in vivo. For this purpose, antibody producing hybridomas are inoculated intraperitoneally into syngeneic mammals, and after 1 to 3 weeks, the antibodies are isolated from the ascites fluid of those mammals. For example, hybrid cells originating from BALB/c mice are injected intraperitoneally into BALB/c mice that have previously been pretreated intraperitoneally with a hydrocarbon such as 2,6, 10,14-tetramethylpentadecane (pristane) to irritate the peritoneal cavity, and, after 8 to 10 days, ascites fluid is withdrawn from these animals.

The monoclonal antibodies produced in vitro or in vivo may be purified using various methods, for example, gel filtration chromatography, ion-exchange chromatography, DEAE-cellulose chromatography or affinity chromatography. Optionally, selected proteins in the culture supernatants or ascites fluid, including the desired monoclonal antibodies, may be precipitated using specific concentrations of ammonium sulphate or the like before being subjected to chromatography.

If desired, derivatives of the monoclonal antibodies produced either in vitro or in vivo may be prepared.

Derivatives of monoclonal antibodies according to the invention include, for example, fragments, such as Fab, Fab' or F(ab')$_2$ fragments, that retain their specificity for the antigenic determinants of the antigen of interest, and radioactively labelled monoclonal antibodies which are labelled, for example, with radioactive iodine ($^{125}$I, $^{131}$I), carbon ($^{14}$C), sulphur ($^{35}$S), tritium ($^3$H), $^{112}$In, $^{99m}$Tc or the like, and monoclonal antibodies conjugated with enzymes such as horseradish peroxidase, alkaline phosphatase, $\beta$-D-galactosidase, glucose oxidase, glucoamylase, carbonic anhydrase, acetylcholinesterase, lysozyme, malate dehydrogenase or glucose-6-phosphate dehydrogenase. Additional derivatives include monoclonal antibodies labeled with fluorescent materials such as fluorescein or rhodamine, and monoclonal antibodies labelled with biotin.

Fragments of monoclonal antibodies according to the invention, for example, Fab, Fab' or F(ab')$_2$ fragments, that retain their specificity for the antigenic determinants of the antigen of interest, may be prepared according to generally known methods, for example, by fragmenting monoclonal antibodies by proteolytic digestion with enzymes such as pepsin or papain and/or by cleavage of disulphide bonds by chemical reduction.

Monoclonal antibodies radioactively labelled with iodine ($^{125}$I, $^{131}$I) may be obtained by iodination, for example, with radioactive sodium or potassium iodide after oxidization with a chemical oxidizing agent, such as sodium hypochlorite, chloramine T or the like, or an enzymatic oxidizing agent, such as lactoperoxidase and glucose oxidase. Radioactively labelled monoclonal antibodies according to the invention may also be prepared by adding, to the culture media for the in vitro culturing, in a known manner, radioactively labelled nutrients containing radioactive carbon ($^{14}$C), tritium ($^3$H), sulphur ($^{35}$S) or the like, for example, L-($^{14}$C)-leucine, L-($^3$H)-leucine or L-($^{35}$5)-methionine, and obtaining the monoclonal antibodies as described above.

Enzyme-conjugated monoclonal antibodies according to the invention may be obtained by various generally known methods, for example, by reacting monoclonal antibodies and the desired enzyme after modification with coupling reagents such as aldehydes, carbodiimides, maleimides, imidates, succinimides and pyridyl disulfides. Specific coupling agents include, for example, glutaraldehyde, periodate, N,N'-o-phenylenedimaleimide, N-(m-maleimidobenzoyloxy)-succinimide, N-(3-(2'-pyridyldithio)-propionoxy)-succinimide or the like.

Various enzyme substrates, for example 5-aminosalicyclic acid, O-phenylenediamine, 3,3'-dimethoxybenzidine, and 2,2'-azino-bis-(3)-ethylbenzothiazolin-6-sulphonic acid for horseradish peroxidase and p-nitrophenyl phosphate for alkaline phosphatase, may be used in conjunction with the enzyme-conjugated antibodies.

Other derivatives contemplated by the present invention include, for example, monoclonal antibodies conjugated to a toxin, such as diptheria toxin, ricin or deglycosylated ricin A chain, or attached to some other substance (e.g., a radioisotope) capable of limiting the growth of or destroying cancer cells, for therapeutic purposes, (i.e., for treating cancer). Such derivatives may be prepared using methods generally known in the art. In using these monoclonal antibody derivatives to treat cancer, the monoclonal antibody derivatives may be administered in an appropriate manner (e.g., using a parenteral solution) to various mammalian species known to be subject to cancer, e.g., humans, cats, dogs and the like, in a therapeutically effective amount within the dosage range of about 0.01 to 100 mg/kg/day, preferably about 0.1 to 10 mg/kg/day, on a regimen in single or 2 to 4 divided daily doses. Alternatively, the solution may be continuously administered to supply these dosage amounts. The active substance should be utilized in a solution containing about 1.0 to about 10.0 mg per unit of dosage of the derivatized monoclonal antibody. They may be formulated in a conventional manner along with other physiologically acceptable materials, such as preservatives and stabilizers as called for by accepted pharmaceutical practice.

It should be understood that fragments of the monoclonal antibodies of the present invention may also be labeled with the various materials referred to above, for example, radioisotopes, enzymes, fluorescent materials, biotin, toxins and substances capable of limiting the growth of cancer cells, in the same manner described above for the full length monoclonal antibody molecules. Such variations are included within the scope of the present invention.

It is contemplated that the present invention encompasses all monoclonal antibodies exhibiting the characteristics of the monoclonal antibodies described herein. The monoclonal antibodies described herein belong to the class IgM, and the subclasses IgG$_1$ and IgG$_{2b}$. It is contemplated that antibodies having the patterns of reactivity illustrated herein are included within the scope of the present invention regardless of the immune globulin class or subclass to which they belong. For example, a monoclonal antibody exhibiting the characteristics described herein may be of the subclass IgG$_1$, IgG$_2$a, IgG$_2$b, or IgG$_3$, or of classes IgM, IgA, or of other known Ig classes.

Furthermore, since the hybrid cell line produced from a known mouse myeloma cell line and spleen cells from a known species of immunized mouse cannot be further identified except by reference to the antibody produced by the hybrid cell line, it is contemplated that all hybrid cell lines producing antibodies having the reactivity characteristics described above are included within the scope of the present invention.

The present invention further concerns immunoassay methods utilizing monoclonal antibodies and derivatives thereof which bind *trk* proto-oncogene protein and/or *trk* related oncogene protein for the qualitative and quantitative determination of *trk* proto-oncogene protein and/or *trk* related oncogene protein, especially in a biological sample.

Particularly preferred is a qualitative immunoassay method for detecting the presence of *trk* proto-oncogene protein and/or *trk* related oncogene protein in a sample comprising:

(a) incubating the sample with a monoclonal antibody which binds to the *trk* proto-oncogene protein and/or *trk* related oncogene protein; and

(b) detecting the presence of immune complexes formed by the *trk* proto-oncogene protein and/or *trk* related oncogene protein and the monoclonal antibody.

Additionally preferred is an immunoassay method for quantitatively determining the amount of *trk* proto-oncogene protein and/or *trk* related oncogene protein in a sample comprising:

(a) incubating the sample with a monoclonal antibody which binds to the *trk* proto-oncogene protein and/or *trk* related oncogene protein ;

(b) determining the amount of immune complexes formed by the *trk* proto-oncogene protein and/or *trk* related oncogene protein and the monoclonal antibody; and

(c) correlating the amount of immune complexes formed with the amount of *trk* proto-oncogene protein and/or *trk* related oncogene protein present in the sample.

The immunoassay methods of the present invention may be either *in vivo* or *in vitro* methods.

In the case of *in vivo* imaging methods, a monoclonal antibody or monoclonal antibody fragment which has been labeled with an appropriate detectable imaging moiety, for example, a radioisotope (e.g., $^{131}$I, $^{112}$In, [99m]Tc), a radioopaque substance, or a material detectable by nuclear magnetic resonance, is introduced (e.g., parenterally, subcutaneously or intraperitoneally) into the subject (e.g., a human) to be examined. The size of the subject, and the imaging system used, will determine the quantity of imaging moiety needed to produce diagnostic images. In the case of a radioisotope moiety, for a human subject, the quantity of radioactivity injected will normally range from about 5 to 20 millicuries of technetium-99m. The labelled monoclonal antibody or monoclonal antibody fragment will then preferentially accumulate at the location of cells which contain *trk* proto-oncogene protein and/or trk related oncogene protein. The labelled monoclonal antibody or monoclonal antibody fragment can then be detected using known techniques. For a general discussion of this technological area, see S. W. Burchiel et al. "Immunopharmokinetics of Radiolabeled Antibodies and their Fragments", Chapter 13 in "Tumor Imaging, The Radiochemical Detection of Cancer", editors S. W. Burchiel and B.A. Rhodes, Masson Publishing Inc., (1982).

If desired, in order to reduce antigenicity in humans, genomic DNA fragments encoding the heavy and light chain variable regions of the mouse monoclonal antibodies which bind *trk* proto-oncogene protein and/or *trk* related oncogene protein can be cloned and transferred into expression vectors that can result in the production of human immunoglobulins that retain the specificity of the mouse antibody as described in Sun, L. K. et al., Proc. Natl. Acad. Sci. (USA) 84, 214-218 (1987).

Rearrangement of the *trk* proto-oncogene have been identified with high frequency in human papillary thyroid carcinomas. As will be shown, the monoclonal antibodies of the present invention are able to recognize rearranged *trk* proto-oncogene protein, that is, *trk* related oncogene protein. Thus, the accumulation of labelled monoclonal antibody or monoclonal antibody fragments in a particular location in a subject may indicate the presence of a primary tumor or a metastatic growth.

In the case of *in vitro* methods, the immunoassay method of the present invention may be a radioimmunoassay (RIA) which utilizes, depending on the particular protocol employed, unlabeled or radioactively labeled derivatives of monoclonal antibodies which bind *trk* proto-oncogene protein and/or *trk* related oncogene protein, either alone or in combination. In the case where the monoclonal antibody which binds *trk* proto-oncogene protein and/or *trk* related oncogene protein is unlabeled, a different detectable marker, for example, a radiolabeled antibody which is capable of binding the monoclonal antibody which binds *trk* proto-oncogene protein and/or *trk* related oncogene protein, may be employed. Any of the known modifications of RIA, for example, homogeneous RIA, heterogeneous RIA, competitive RIA, and sandwich RIA, may be employed.

The immunoassay method of the present invention may also be an enzyme immunoassay (EIA) which utilizes, depending on the particular protocol employed, unlabeled or enzyme-labeled derivatives of monoclonal antibodies which bind *trk* proto-oncogene protein and/or *trk* related oncogene protein, either alone or in combination. In the case where the monoclonal antibody which binds *trk* proto-oncogene protein and/or *trk* related oncogene protein is not enzyme labelled, a different detectable marker, for example, an enzyme-labeled antibody capable of binding to the monoclonal antibody which binds *trk* proto-oncogene protein and/or *trk* related oncogene protein, may be employed. Any of the known modifications of EIA, for example, enzyme-linked immunoabsorbent assay (ELISA), may be employed.

The immunoassay method of the present invention may also be other known immunoassay methods, for example, fluorescent immunoassays using antibody conjugates or antigen conjugates of fluorescent substances such as fluorescein or rhodamine, latex agglutination with antibody-coated or antigen-coated latex particles, haemagglutination with antibody-coated or antigen-coated red blood corpuscles, and immunoassays employing an avidinbiotin or strepavidin-biotin detection system.

The particular parameters employed in the *in vitro* immunoassays of the present invention can vary widely depending on various factors such as the concentration of antigen in the sample, the nature of the sample, the type of immunoassay employed and the like. Optimal conditions can be readily established by those of ordinary skill in the art. The amount of antibody which binds *trk* proto-oncogene and/or *trk* related oncogene protein is typically selected to give 50% binding of detectable marker in the absence of sample. If purified antibody is used as the antibody source, the amount of antibody used per assay will generally range from about 1 ng to about 100 ng. Typical assay conditions include a temperature range of about 4° C to about 45° C, preferably about 25° C, a pH value range of about 5 to 9, preferably about 7, and an ionic strength varying from that of distilled water to that of about 0.2 M sodium chloride, preferably about that of 0.15 M sodium chloride. Times will vary widely depending upon the nature of the assay, and generally range from about 0.1 minute to about 24 hours. A wide variety of buffers, for example PBS, may be employed, and other reagents such as salt to enhance ionic strength, proteins such as serum albumins, stabilizers, biocides and non-ionic detergents may also be included.

The presence of *trk* related oncogene protein in a biological sample may indicate the presence of cancer in the subject from which the sample is obtained.

It should be understood that fragments of the monoclonal antibodies of the present invention may be utilized in the immunoassay methods of the present invention, and that such variations are included within the scope of the present invention.

The monoclonal antibodies of the present invention may also be used to purify *trk* proto-oncogene protein and/or *trk* related oncogene protein. Briefly, monoclonal antibodies which bind *trk* proto-oncogene protein and/or *trk* related oncogene protein may be bound to a substrate (e.g., a solid support such as Protein-A Sepharose), and contacted with a preparation (e.g., a solution) containing *trk* proto-oncogene protein and/or *trk* related oncogene protein under conditions allowing the monoclonal antibodies to bind the *trk* proto-oncogene protein and/or *trk* related oncogene protein. Any unbound material is separated from the immobilized monoclonal antibodies, and the bound *trk* proto-oncogene protein and/or *trk* related oncogene protein recovered from the monoclonal antibodies (e.g., by elution with a suitable eluant) to yield purified *trk* proto-oncogene protein and/or *trk* related oncogene protein.

The purified *trk* proto-oncogene protein may then be used for drug screening, in particular, to identify drugs which bind to or inhibit the function of *trk* oncogene protein but not *trk* proto-oncogene protein. The monoclonal antibodies of the present invention may also be used in the identification of *trk* proto-oncogene protein and/or *trk* related oncogene protein in crude cellular extracts and when expressed in mouse and human cells.

The monoclonal antibodies of the present invention can also be used to aid in the identification of the ligand of the proto-*trk* receptor.

The following examples are further illustrative of the present invention. These examples are not intended to limit the scope of the present invention and provide further understanding of the invention.

Example 1

Cell Culture

The mouse myeloma (plasmacytoma) cell line, Sp2/0-Ag14, deficient in hypoxanthine guanine phosphoribosyl transferase (HGPRT) was employed. This cell line is available from the American Type Culture Collection (Rockville, MD) and the National Institute of General Medical Sciences Human Genetic Mutant Cells Depository (Camden, NJ). Sp2/0 cells and selected hybridomas were cultured at 37°C in a humidified 8% $CO_2$ atmosphere in Dulbecco's Modified Eagles Medium (DMEM) with high glucose (4.5 g/liter) supplemented with 10% fetal calf serum and L-glutamine (0.3 mg/ml). DMEM and L-glutamine were obtained from Gibco Laboratories (Life Technologies Inc., Grand Island, NY) and fetal calf serum was obtained from Hyclone Laboratories Inc., Logan, Utah. All other medium constituents were obtained from Sigma Chemical Company, St. Louis, MO unless otherwise indicated. After the fusion, cells were grown in HY medium (HY medium : DMEM supplemented with 10% NCTC 109 (Gibco), 15% fetal calf serum, 0.2 units bovine insulin/ml, 0.45 mM pyruvate, 1 mM oxaloacetate and 0.1% glutamine) containing hypoxanthine (0.1 mM), aminopterin (0.1 $\mu$m) and thymidine (0.016 mM).

Example 2

Preparation of Sera-Positive Animals

Sera-positive animals were produced by the subcutaneous immunization of *trk* proto-oncogene/pSV2neo transfected NIH3T3 cells. This cell line, E25-4-27, previously described by Martin-Zanca, et al. "Molecular and biochemical characterization of the human *trk* proto-oncogene", Mol. Cell. Biol., 9 : 24-33 (1989), was cultured at 37°C in a humidified 8% $CO_2$ atmosphere in Dulbecco's Modified Eagles Medium (DMEM) with high glucose (4.5 g/liter) supplemented with 10% calf serum, L-glutamine (0.3 mg/ml) and geneticin (G418) (500 $\mu$g/ml). Cells used as the immunogen were grown to near confluency and scraped from flasks. The cells were washed in Hanks Balanced Salt Solution and prepared for injection into the groin region of 6-week old NIH/Swiss mice. Tumors of 2 mm diameter were seen as early as day 5 following the injection of 5-10 x $10^6$ cells. In some mice peak tumor growth was obtained at day 10-15 prior to tumor regression. In other mice having received the same immunogen, tumors continued to grow until sacrifice at day 22-25.

Example 3

Production of Hybridomas

Tumor-bearing animals were sacrificed and the spleens removed. Spleens were removed not only from mice where the tumor mass regressed but also from mice where the tumor mass was continuing to grow. Fusions were performed according to a modification of the method of Kohler and Milstein [Nature, 256, 495-497 (1975)] using Koch-light polyethylene glycol 4000. Spleen cells from the selected animals were collected by perfusion with medium introduced by a syringe and erythrocytes lysed in cold 0.17 M $NH_4Cl$. The collected cells were counted, mixed at a ratio of $10^8$ spleen cells to 2 x $10^7$ myeloma cells in a round-bottomed tube. The cell mixture was washed in medium free of serum by centrifugation. All supernatant liquid was removed by suction and the pellet loosened. 0.5 mls of PEG solution (30% polyethylene glycol, 5% dimethylsulfoxide in medium without serum) was slowly added to the pellet. The cells were maintained in the PEG solution for 8 minutes during which time they were pelleted at 1000 rpm for 5-6 minutes. Medium without serum (5 mls) was slowly added to disperse the pellet followed by the addition of 5 mls of HY medium containing 15% fetal calf serum. The cells were pelleted and evenly resuspended in HY medium spplemented with hypoxanthine, aminopterin and thymidine resulting in a cell suspension of 1.5 x $10^6$ cells per ml. Cells were then plated out in 96-well microtiter plates (100 $\mu$l/well) and placed in a humidified $CO_2$ incubator at 37°C. The wells were refed 6-7 days later. Clones growing in selection medium in microtiter plate wells were identified by examining the plate macroscopically using an inverted mirror stand. Medium from wells containing these clones was tested for the presence of specific antibody by enzyme linked immunosorbent assay (ELISA; see Example 5).

Example 4

Expansion of Antibody Producing Hybridomas

Hybridomas producing specific antibody as demonstrated by ELISA were expanded by standard cell culture techniques and grown for several passages in HY media supplemented with hypoxanthine and thymidine. Cells were adapted to Dulbecco's Modified Eagles medium supplemented with 10% calf serum and glutamine (0.1%). Hybridomas of interest were subcloned by limiting dilution in freshly prepared HY media; clones were screened by ELISA. Subcloned hybridomas and the original lines were cryopreserved by standard techniques using a freezing mixture of 95% calf serum with 5% dimethylsulfoxide.

Antibody was collected in cell culture supernatant by accumulating antibody from densely growing cultures. In addition to cell culture methods, hybridomas were also grown in the peritoneal cavity of nude (*nu/nu*) mice. Mice were injected intraperitoneally with 0.5 ml of pristane (2, 6, 10, 14-tetramethyldecanoic acid, Aldrich Chemical Company, Inc., Milwaukee, WI) at least 10 days prior to injection with the hybridoma line of interest. Hybridomas (approx 4 x $10^6$ cells) shown to have activity in an ELISA prior to injection were inoculated intraperitoneally into the pristane treated nude mice to produce ascites fluid. Ascites fluid was removed from the mice after 7-10 days and clarified by centrifugation at 35000 rpm in a Beckman 50 Ti rotor at 6°C. Clarified ascites was stored at 4°C with 0.02% sodium azide or frozen in aliquots at -70°C.

Example 5

Enzyme Linked Immunosorbent Assay (ELISA)

Sera from immunized mice and media collected from wells containing HAT selected hybridomas were tested for the presence of antibodies recognizing the proto-*trk* protein. The antigens used for screening were a subcellular fraction prepared from both the *trk* proto-oncogene transfected cell line E25-4-27 and the partner cell line E25-3-2, NIH3T3 transfected with pSV2neo alone. The cell lines E25-4-27 and E25-3-2 were grown as described above in Dulbecco's modified Eagles medium supplemented with calf serum, glutamine and geneticin (500 $\mu$g/ml). Cells used for subcellular fractionation were grown to approximately 75% confluency and collected by scraping. Cells were washed several times in Hanks Balanced Salt Solution. Pelleted cells were resuspended in fractionation buffer [20 mM HEPES pH 7.4, 1 mM EDTA, 1 mM magnesium chloride, 1 mM dithiothreitol, 1 mM phenyl methylsulfonyl fluoride (PMSF)] and sonicated. After centrifugation at 1000 x g for 10 minutes, the supernatant was centrifuged at 150,000 x g for 30 minutes at 4°C. The supernatant was discarded and the remaining pellet washed in fractionation buffer, sonicated and centrifuged at 150,000 x g for 30 minutes at 4°C. The supernatant was discarded and the pellet solubilized in Staph A buffer (10 mM sodium phosphate [pH 7.0], 1% Triton-X100, 0.1% sodium dodecyl sulfate, 0.1% sodium azide, 100 mM sodium chloride, 0.5% sodium deoxycholate). This fraction is called the p100 fraction. Protein concentration was determined using the Bio-Rad Protein Assay Kit (Bio-Rad, Richmond, CA). p100 fractions were aliquoted and frozen immediately on dry ice/methanol and placed at 70°C for long-term storage.

The p100 fraction was diluted in phosphate buffered saline (6 $\mu$g/ml) and adsorbed to polystyrene plates for the ELISA procedure. 50 $\mu$l of this solution was added per well of a distilled water-rinsed Nunc Immuno Plate IF 96-well plate (A/S Nunc, Kamstrup, Denmark) and sealed. After incubation overnight at 4°C, the plates were rinsed six times with PBS-Tw20 (Phosphate buffered saline with 0.05% Tween 20). Undiluted media (50 $\mu$l) or diluted mouse sera (50 $\mu$l) were incubated for two hours at room temperature. Unbound mouse antibodies were removed by washing the plates six times with the PBS-Tw20 wash. After washing, 100 $\mu$l of diluted alkaline phosphatase conjugated-goat anti-mouse immunoglobulin IgG (heavy and light chain) (Jackson Immunoresearch Laboratories, Inc., West Grove, PA) was added per well for a period of two hours at room temperature. Antisera was diluted to 1:3000 in ELISA buffer consisting of PBS-Tw20 supplemented with 20.4 g sodium chloride per liter, 0.29 g EDTA per liter and 0.2% peroxidase-free bovine serum albumin. Removal of unbound conjugate was accomplished by extensive washings with PBS-Tw20 followed by several washes with Tris-buffered saline, pH 7.5 (0.05 M Tris-hydrochloride, 0.15 M sodium chloride). Bound conjugate was detected by visualization using an alkaline phosphatase ELISA amplification kit prepared by Bethesda Research Laboratories/Life Technologies, Inc., Gaithersburg, MD. Substrate and Amplifier reagent were prepared as directed by the manufacturer using the appropriate substrate and amplifier diluents. 50 $\mu$l of the substrate solution was added per well and the reaction allowed to proceed for 20 minutes at room temperature with shaking on a Dynatech plate shaker. Fifty microliters of amplifier reagent was added and incubated fifteen minutes at room temperature before the reaction was stopped by the addition of 50 microliters of 0.3 M sulfuric acid. The reaction products measured by optical densities were quantitated on a Titertek Multiskan PLUS microtiter plate reader (Flow Laboratories, Inc., McLean, VA) at 492 nm.

Example 6

Determination of Immunoglobulin Class/Subclass

To determine the immunoglobulin class/subclass of the hybridomas, cell culture supernatant containing antibody was assayed in an ELISA format. Affinity purified goat anti-mouse immunoglobulin (heavy and light chain) (Organon Teknika-Cappel, Malvern, PA) was diluted 1:800 in 0.05 M sodium carbonate buffer, pH 9.6. 50 $\mu$l of this solution was added per well of a distilled water- rinsed Nunc Immuno Plate IF and incubated 2 hours at room temperature or overnight at 4°C. Unadsorbed antibody was removed by washing with PBS-Tw20. Fifty microliters of antibody-containing supernatant or diluted myeloma ascites controls were added and incubated for a period of two hours at room temperature. Unbound antibody was removed by extensive washings. Alkaline phosphatase conjugated goat anti-mouse immunoglobulin class and subclass specific reagents (FisherBiotech, Orangeburg, NY) were diluted 1:500 in PBS-Tw20 buffer with 0.2% bovine serum albumin. One hundred microliters of each conjugate was added to a well previously incubated with the anti-mouse Ig and the mouse antibody of interest. The alkaline phosphatase conjugate was incubated for two hours at room temperature and the plates washed four times with PBS-Tw20 followed by four washes of Tris buffered saline (0.05 M Tris HCl, 0.15 M NaCl pH 7.5). Alkaline phosphatase activity was visualized using p-nitrophenyl phosphate (1 mg/ml) diluted in alkaline phosphatase buffer (10 mM diethanolamine containing 0.5 mM MgCl). The product of the reaction was scored by eye or quantitated at 405 nm with a Titertek Multiskan PLUS microtiter plate reader.

Example 7

Monoclonal Antibody Purification

About 2 to 4 mls of ascites fluid produced from the appropriate hybridoma was diluted 1:1 with Binding Buffer supplied by Bio Rad in their Bio-Rad Affi-Gel Protein A MAPS II Kit (Bio-Rad Laboratories, Richmond, CA) and applied to a 1 cm x 5 cm Affi-Gel Protein A column pre-equilibrated with Binding Buffer. Diluted ascites was applied at a rate of 0.2-0.3 ml/minute. Effluent was collected and reapplied to the column prior to washing. The elution of bound antibody was carried out using the Bio-Rad Elution Buffer at a rate of 0.5 ml/minute. Fractions were neutralized immediately by the addition of 1 M Tris HCl, pH 9.0. Elution of immunoglobulin was monitored by determining the absorbance of each sample at 280 nm. Samples showing significant protein levels were pooled and dialyzed against PBS at 4°C. The protein concentration was calculated using an extinction coefficient for immunoglobulin of E = 1.4 $cm^2$/mg. Antibody activity was determined using the ELISA described above. Purity of the immunoglobulin was determined by agarose gel electrophoresis by the Paragon system (Beckman Instruments, Fullerton, CA) and standard sodium dodecyl sulfate-polyacrylamide gel electrophoresis.

Example 8

Analytical Methods

Sodium dodecyl sulfate-polyacrylamide gel electrophoresis (SDS-PAGE) was performed using gels and buffers described by Laemmli, U.K., "Cleavage of Structural Proteins During Assembly of the Head of Bacteriophage T$_4$", Nature 227:680-685 (1970). Samples were prepared in a buffer containing $\beta$-mercaptoethanol and SDS, heated and subjected to electrophoresis.

Immunoblotting was performed on p100 fractions prepared from the NIH3T3 transfected proto-*trk* positive and negative cell lines described in Example 5 and electrophoresed by SDS-PAGE. The proteins were electrophoretically transferred to nitrocellulose sheets according to the method of Towbin, H.T., Staehelin, T. and Gordon, J., "Electrophoretic transfer of protein from polyacrylamide gels to nitrocellulose sheets: Procedure and some applications", Proc. Natl. Acad. Sci. (USA), 76:4350-4354 (1979). The nitrocellulose membrane was blocked with 5% BSA (fraction V) prepared in Tris-buffered saline (0.02 M Tris HCl, 0.5 M NaCl, pH 7.5) for 30-60 minutes at room temperature with agitation. The blocked nitrocellulose membrane was then either cut into strips for incubation with antibody containing supernatant or diluted antiserum or placed into a Miniblotter manifold (Immunetics, Cambridge, MA) and appropriate antibodies added to the channels. Incubation with the first antibody was carried out at room temperature with agitation for 1-2 hours. Several washes with TBS were done to remove unbound antibody. An alkaline phosphatase conjugated affinity purified goat anti-mouse IgG (heavy and light chains) reagent (Jackson ImmunoResearch Laboratories, Inc., West Grove, PA) was diluted 1:3000 in TBS containing 0.05% Tween 20 and 1% gelatin and added to the blot and incubated 1-2 hours at room temperature with agitation. Again, the blot was extensively washed with TBS before addition of substrate. The sites of enzyme binding were detected by the use of BCIP/NBT (5-bromo-4-chloro-3-indolyl phosphate/nitroblue tetrazolium) substrate supplied in kit form from Kirkegaard and Perry (Gaithersburg, MD).

Example 9

Radioimmunoprecipitation of *trk*, proto-*trk* and *trk*-related proteins

Subconfluent cell lines in 100-mm dishes were washed once with Hanks Balanced Salt Solution (HBSS), once with methionine-free Dulbecco's modified Eagles medium (DMEM) and incubated for one hour in 3.5 mls of methionine-free DMEM supplemented with 10% dialyzed calf serum. This medium was aspirated and fresh medium containing 0.050 mCi [$^{35}$S] Tran$^{35}$Slabel [ICN; a mixture of radiolabeled [$^{35}$S] methionine (85%) and radiolabeled [$^{35}$S] cysteine (15%)] was added and incubated for four hours. To examine non-glycosylated radiolabeled proteins, cell lines were labeled as described above with the addition of the antibiotic tunicamycin at 5 $\mu$g/ml. The labelling medium was aspirated and the cells rinsed with HBSS. The cells were lysed on ice for 15 minutes with 1.0 ml of Staph A buffer (See Example 5) containing 1 mM PMSF and 2.5 TIU (Trypsin Inhibitor Units) of aprotinin per milliliter. The cell extract was collected and centrifuged at 15,000 x g at 4$^{\circ}$C. The supernatant was removed, trichloroacetic acid counts determined and used immediately or frozen at -70$^{\circ}$C. The extract was incubated overnight at 4$^{\circ}$C with 2 microliters of appropriate antiserum or 150 microliters of dense supernatant containing monoclonal antibodies. Protein A-Sepharose CL-4B (Pharmacia, Uppsala, Sweden) was prepared to 30 mg/ml; ProA-Seph was coated with goat anti-mouse immunoglobulin (heavy and light chains) (Organon Teknika-Cappel) (2 mls per 150 mg ProA-Seph) to enhance binding of all mouse subclasses. 200 microliters of Protein A-Sepharose was added to each reaction vessel containing extract plus antibody and incubated for one hour at 4$^{\circ}$C. The samples were washed three times with freshly prepared Staph A buffer and reducing sample buffer added to the immunocomplexes and heated to 90$^{\circ}$C for five minutes before application to SDS-containing acrylamide gels as described in Example 8. The gels were fixed and processed for autoradiography. Radiolabeled markers were added to determine the relative molecular weights of immunoprecipitated protein.

Example 10

Identification and Characterization of Monoclonal Antibodies

Tumor-bearing mice had developed significant levels of antibody as determined by sera testing in immunoprecipitation experiments. These mice were producing antibodies that were able to immunoprecipitate the 140 kd and 110 kd glycoproteins of the *trk* proto-oncogene. The spleens of responding animals were removed and fused with the myeloma line as described above. In a total of three fusions, over 40% of the wells plated (total 2150 wells) produced HAT resistant clones distinguishable by light microscopy. Antibody producing hybridomas were identified by the ELISA procedure described in Example 5. All supernatants were screened against the p100 fraction prepared from the E25-4-27 line (+ *trk* proto-oncogene) and the E25-3-2 (negative control). Supernatants yielding a positive reaction with the extract prepared from the E25-4-27 line and non-reactive with the extract from the identical line not expressing the *trk* proto-oncogene were saved and assumed to be specific for the proto-*trk* glycoprotein. Eleven clones were identified with this property from the screening of over 800 supernatants. All eleven clones continued to demonstrate such activity following expansion and subcloning. The antibody hybridomas were named with the prefix TTM or TUM and a corresponding number (i.e. TTM7-4) with subclones denoted by a suffix of .1-.n. The heavy chain isotype and light chain class of the corresponding antibodies (prefixed MabTTM or MabTUM; antibodies produced by subclones denoted by .1-.n as above) were determined by ELISA as previously described in Example 6. This data is listed in Table 1.

All of the monoclonal antibodies were analyzed to determine which specific proteins present in the E25-4-27 extract were reactive with the monoclonal antibodies. Immunoblotting analysis as described in Example 8 with extracts of E25-4-27 and E25-3-2 revealed that none of these antibodies recognized the immunoblotted proteins in contrast to rabbit antisera prepared to the 14 terminal amino acids of the tyrosine kinase domain which detected both the 140 Kd and 110 Kd proto-oncogene polypeptides. Since this technique involved denaturation of the antigen, recognition by these monoclonal antibodies suggested that the epitopes recognized the protein in the native, undenatured configuration. The ability of the proto-*trk* monoclonal antibodies to recognize specific proteins when free in solution was confirmed by immunoprecipitation protocols using *in vitro* labelled cell extracts. All of the antibodies immunoprecipitate solely the 140 Kd and the 110 Kd proto-*trk* glycoproteins from the *trk* proto-oncogene containing cell line, E25-4-27. The best antibodies for immunoprecipitation are TTM6-13 and TTM7-4.

The eleven monoclonal antibodies were analysed for the degree of cross-reactivity with alpha-2-macroglobulin, β-galactosidase and bovine serum albumin. The purified proteins were adsorbed to polystyrene plates as described in Example 5. None of the antibodies gave specific reactions with any protein except the p100 fraction from the E25-4-27 cell line.

Example 11

Properties of Proto-*trk* Monoclonal Antibodies

Since it had been determined that the panel of *trk* proto-oncogene monoclonal antibodies were specific for the 140 and 110 kilodalton proto-oncogene glycoproteins, proteins known to be related to the *trk* proto-oncogene were analyzed by immunoprecipitation as described in Example 9. None of the monoclonal antibodies described immunoprecipitated the *trk* oncogene protein of 70 Kd or the *trk* B (*trk* -related murine protein) glycoproteins of 145 and 120 Kd. Immunoprecipitation of proteins from tunicamycin-treated E25-4-27 expressing the *trk* proto-oncogene, demonstrated that the monoclonal antibodies did not bind and precipitate the unglycosylated *trk* proto-oncogene. In all of these cases, the expressed proteins were made in murine cell lines. The human osteosarcoma cell line, HOS, has also been used for expression of the *trk* proto-oncogene although levels of this protein are significantly less than in the NIH3T3 transfectants. All of the monoclonal antibodies described are able to immuno-precipitate the human *trk* proto-oncogene (140, 110 Kd) when expressed in human cells.

Additional *trk* oncogenes described by Oskam et al. in "Frequent generation of oncogenes by *in vitro* recombination of *TRK* protooncogene sequences", Proc. Natl. Acad. Sci. (USA) 85 : 2964-2968 [1988] and Coulier et al. in "Human *trk* oncogenes activated by point mutation, in-frame deletion and duplication of the tyrosine kinase domain", submitted Molec. Cell. Biol. [1990] were analyzed by immuno-precipitation of the radiolabelled extracts prepared from the cell lines expressing the *trk* -related proteins. The results of immunoprecipitations of the specific *trk* -related oncogene proteins (molecular weights given for the known products) are summarized in Table 2. None of the antibodies recognize the protein product expressed by the *trk* 6 mutant, a non-glycosylated 62 Kd protein. Monoclonal antibodies TTM1-8•1, TTM6-13•1, TTM6-46•6, TTM6-50•2, TTM7-4•1, TTM7-41•1, TTM9-9•2 and TTM9-12•1 immunoprecipitate all expected *trk* -related oncogene proteins. Monoclonal antibodies TUM5-47•5 and TUM7-9•31 immunoprecipitate the *trk* -related proteins from some of the transforming cell lines.

Example 12

Immunochemical localization of the *trk* proto-oncogene with monoclonal antibodies

The group of monoclonal antibodies were studied for the ability to localize the intracellular expression of the *trk* proto-oncogene. Immunoperoxidase staining was performed on E25-4-27 (*trk* proto-oncogene positive) and E25-3-2 (control) cells that were grown to approximately 50% confluency in 8-chambered LabTek tissue culture slide (Miles Scientific). The slides were briefly washed in 85% phosphate buffered saline with calcium and magnesium salts added. A 1:1 mixture of absolute methanol and acetone was added to the chambers and incubated for 10 minutes at room temperature. The fixative was removed and the wells washed three times with PBS as described above. Non-specific binding was blocked by the incubation of 5% bovine serum albumin in PBS for 45 minutes at room temperature. Blocking solution was aspirated, wells washed and cell culture supernatant containing monoclonal antibody was added and incubated overnight at 4°C. Supernatant was aspirated, wells washed with PBS and incubated for three hours at room temperature with a 1:200 dilution of horseradish peroxidase conjugated goat anti-mouse immunoglogulin (Organon Teknika-Cappel, Malvern, PA) prepared in PBS. The wells were washed three times with PBS prior to the addition of diaminobenzidene (DAB) containing-substrate (0.3 mg/ml DAB in PBS with 30% hydrogen peroxide added at 1 microliter/mls). All of the monoclonal antibodies showed an intense staining reaction localized in the *trk* -transfected E25-4-27 cells. Monoclonal antibody TUM5-47 and subclones demonstrated a greatly reduced staining in the control E25-3-2 cells.

## Table 1

### Immunochemical Characteristics of *trk* Proto-oncogene Monoclonal Antibodies

| Monoclonal Antibody | Isotype | | Immunoreactivity | |
|---|---|---|---|---|
| | Light chain | Heavy chain | Immunoblot | Immunoppt 140/110 Kd |
| MabTTM1-8 | kappa | gamma 1 | - | + |
| MabTTM6-13 | kappa | gamma 1 | - | + |
| MabTTM6-46 | kappa | gamma 2b | - | + |
| MabTTM6-50 | kappa | gamma 1 | - | + |
| MabTTM7-4 | kappa | gamma 1 | - | + |
| MabTTM7-41 | kappa | gamma 1 | - | + |
| MabTTM9-9 | kappa | gamma 2b | - | + |
| MabTTM9-12 | kappa | gamma 2b | - | + |
| MabTUM5-47 | kappa | mu | - | + |
| MabTUM7-9 | kappa | gamma 1 | - | + |

EP 0 471 205 A1

Table 2

PROTO-trk MONOCLONAL ANTIBODY IDENTIFICATION OF trk-RELATED
ONGOGENE PRODUCTS

| Mab | trk 5 (gp120/95) | trk 35 (gp83) | trk 9 (gp150/130) | trk 14 (gp175/145) | trk 15 (gp89) | trk 37 (gp145) | trk 36 (gp107/103 | trk 6 (p62) |
|---|---|---|---|---|---|---|---|---|
| MabTTM 1-8.1 | +/+ | + | +/+ | +/+ | + | + | +/+ | - |
| MabTTM 6-13.1 | +/+ | + | +/+ | +/+ | + | + | +/+ | - |
| MabTTM 6-46.6 | +/+ | + | +/+ | +/+ | + | + | +/+ | - |
| MabTTM 6-50.2 | +/+ | + | +/+ | +/+ | + | + | +/+ | - |
| MabTTM 7-4.1 | +/+ | + | +/+ | +/+ | + | + | +/+ | - |
| MabTTM 7-41.1 | +/+ | + | +/+ | +/+ | + | + | +/+ | - |
| MabTTM 9-9.2 | +/+ | + | +/+ | +/+ | + | + | +/+ | - |
| MabTTM 9-12.1 | +/+ | + | +/+ | +/+ | + | + | +/+ | - |

13

Table 2 (continued)

PROTO-trk MONOCLONAL ANTIBODY IDENTIFICATION OF trk-RELATED ONGOGENE PRODUCTS

| Mab | trk 5 (gp120/95) | trk 35 (gp83) | trk 9 (gp150/130) | trk 14 (gp175/145) | trk 15 (gp89) | trk 37 (gp145) | trk 36 (gp107/103) | trk 6 (p62) |
|---|---|---|---|---|---|---|---|---|
| MabTUM 5-47.5 | -/- | - | +/+ | +/+ | - | + | -/- | - |
| MabTUM 7-9.31 | -/- | - | +/+ | +/+ | + | + | -/- | - |
| antisera 42-3 | +/+ | + | +/+ | +/+ | + | + | +/+ | + |

## Claims

1. A hybrid cell line that produces a monoclonal antibody which binds *trk* proto-oncogene protein and/or *trk* related oncogene protein.

14

2. The hybrid cell line according to Claim 1 designated TTM7-41, or subclones derived therefrom.

3. The hybrid cell line with the identifying characteristics of the hybrid cell line according to Claim 1 designated TTM1-8, TTM6-13, TTM6-46, TTM6-50, TTM7-41, TTM9-9, TTM9-12, TUM5-47, TUM7-9 or TUM10-14, or subclones derived therefrom.

4. The monoclonal antibody secreted by the hybrid cell line according to Claims 2 or 3.

5. The monoclonal antibody secreted by the hybrid cell line according to Claim 1.

6. The monoclonal antibody according to Claim 5 selected from the group consisting of IgG or IgM.

7. A fragment of the monoclonal antibody according to Claim 5.

8. The monoclonal antibody according to Claim 5 designated MabTTM7-41.

9. The monoclonal antibody with the identifying characteristics of the monoclonal antibody according to Claim 5 designated MabTTM1-8, MabTTM6-13, MabTTM6-46, MabTTM6-50, MabTTM7-4, MabTTM7-41, MabTTM9-9, MabTTM9-12, MabTUM5-47, MabTUM7-9 or MabTUM10-14.

10. The monoclonal antibody according to Claims 5, 7, 8 or 9 which has been derivatized.

11. The monoclonal antibody according to Claim 10 which has been labelled with a radioisotope.

12. The monoclonal antibody according to Claim 10 which has been conjugated to an enzyme.

13. The monoclonal antibody according to Claim 10 which has been conjugated with a toxin.

14. The monoclonal antibody according to Claim 10 which has been derivatized with a substance capable of limiting the growth of or destroying cancer cells.

15. The monoclonal antibody according to Claims 5, 7, 8 or 9 which has been substantially purified.

16. An immunoassay method for detecting the presence of *trk* proto-oncogene protein and/or *trk* related oncogene protein in a sample comprising:
    (a) incubating the sample with a monoclonal antibody of fragment thereof which binds to the *trk* proto-oncogene protein and/or *trk* related oncogene protein; and
    (b) detecting the presence of immune complexes formed by the *trk* proto-oncogene protein and/or *trk* related oncogene protein and the monoclonal antibody or fragment thereof.

17. An immunoassay method for quantitatively determining the amount of *trk* proto-oncogene protein and/or *trk* related oncogene protein in a sample comprising:
    (a) incubating the sample with a monoclonal antibody or fragment thereof which binds to the *trk* proto-oncogene protein and/or *trk* related oncogene protein;
    (b) determining the amount of immune complexes formed by the *trk* proto-oncogene protein and/or *trk* related oncogene protein and the monoclonal antibody or fragment thereof; and
    (c) correlating the amount of immune complexes formed with the amount of *trk* proto-oncogene protein and/or *trk* related oncogene protein present in the sample.

18. The immunoassay method according to Claims 16 or 17 which is a radioimmunoassay.

19. The immunoassay method according to Claims 16 or 17 which is an enzyme immunoassay.

20. Use of the monoclonal antibody according to Claims 13 or 14 for preparing a medicament for treating cancer.

21. A composition for treating cancer comprising a therapeutically effective amount of the monoclonal antibody according to Claims 13 or 14 and a pharmaceutically acceptable carrier thereof.

**22.** A method for the purification of *trk* proto-oncogene and/or *trk* related oncogene protein comprising:

(a) contacting a preparation containing *trk* proto-oncogene protein and/or *trk* related oncogene protein to monoclonal antibodies which bind *trk* proto-oncogene protein and/or *trk* related oncogene protein bound to a substrate;

(b) allowing the *trk* proto-oncogene protein and/or *trk* related oncogene protein to bind to the substrate-bound monoclonal antibodies;

(c) separating any unbound material from the substrate-bound monoclonal antibodies;

(d) recovering the *trk* proto-oncogene protein and/or *trk* related oncogene protein from the substrate-bound monoclonal antibodies.

**23.** Use of a monoclonal antibody or fragment thereof which binds *trk* proto-oncogene protein and/or *trk* related oncogene protein bound to an imaging moiety for preparing a formulation for detecting cancer in a subject by means of the signal produced by the imaging moiety.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y,D | MOLECULAR AND CELLULAR BIOLOGY vol. 9, no. 1, January 1989, WASHINGTON DC, USA pages 24 - 33; D. MARTIN-ZANCA ET AL.: 'Molecular and biochemical characterization of the human trk proto-oncogene ' * page 28, right column, line 2 - page 29, left column, line 3 * * page 30, left column, line 25 - right column, line 22 * * abstract * | 1-23 | C12N5/20 C12P21/08 G01N33/574 G01N33/577 A61K47/48 A61K43/00 A61K39/395 C07K3/18 A61K49/02 |
| Y | WO-A-8 706 940 (THE REGENTS OF THE UNIVERSITY OF CALIFORNIA) * claims * | 1-23 | |
| X,P | ONCOGENE vol. 6, no. 5, May 1991, BASINGSTOKE, GB pages 819 - 824; K. EAGER: 'Molecular characterization of human trk proto-oncogene product monoclonal antibodies. ' * the whole document * | 1-23 | TECHNICAL FIELDS SEARCHED (Int. Cl.5) C07K C12P A61K G01N |

The present search report has been drawn up for all claims

| Place of search | Date of completion of the search | Examiner |
|---|---|---|
| THE HAGUE | 27 AUGUST 1991 | NOOIJ F.J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)